# EUROPÄISCHE PATENTSCHRIFT

(11) **EP 0 651 995 B1**
(45) Veröffentlichungstag und Bekanntmachung des Hinweises auf die Patenterteilung: **09.02.2000**
(21) Anmeldenummer: 94117613.3
(22) Anmeldetag: 08.11.1994
(51) Int. Cl.: A61K 9/107

(54) **Cyclosporin(e) enthaltende o/w Emulsion zur oralen Verabreichung**
Cyclosporin(s) containing O/W emulsion for oral administration
Emulsion H/E contenant de la cyclosporine pour administration orale

(30) Priorität: 08.11.1993 DE 4338086
(43) Veröffentlichungstag der Anmeldung: 10.05.1995
(73) Patentinhaber: Dietl, Hans, Dr., 83043 Bad Aibling (DE)
(72) Erfinder: Dietl, Hans, Dr., 83043 Bad Aibling (DE)
(74) Vertreter: Reinhard - Skuhra - Weise & Partner

(56) Entgegenhaltungen:
- EP-A- 0 041 772
- EP-A- 0 391 369

## Beschreibung

Diese Erfindung betrifft eine neue, Cyclosporin(e) enthaltende pharmazeutische Zubereitung zur oralen Applikation, ein Herstellungsverfahren für diese pharmazeutische Zubereitung und ihre Verwendung zur oralen Applikation.

Cyclosporine sind von Wissenschaftlern der Sandoz AG, Basel, entdeckte cyclische Oligopeptide aus niederen Pilzen. Insbesondere Cyclosporin A bzw. Cyclosporin G werden als Immunsuppressiva, insbesondere bei Organ-Transplantationen, eingesetzt. Weiterhin bevorzugt wird das Cyclosporin-Derivat "SDZ IMM 125", ein Hydroxyethylderivat von D-Serin-8-Cyclosporin. Auch die Anwendung bei anderen Erkrankungen, z. B. Diabetes und Psoriasis sowie zahlreichen Autoimmunerkrankungen (z. B. rheumatische Arthritis, endogene Uveitis etc.), wurde beschrieben.

Aus Pilzen wird Cyclosporin A durch Säulenchromatographie über Kieselgel als weißes amorphes Pulver gewonnen, es kristallisiert aus Aceton in weißen Nadeln mit einem Schmelzpunkt von 148 - 151°C. Neben dem Cyclosporin A sind zahlreiche andere Cyclosporine bekannt, die von Cyclosporin A bis Cyclosporin Z reichen (Römpps Chemie Lexikon, 9. Auflage, Seiten 841 - 843). Auch halbsynthetische Derivate des Cyclosporins sind bekannt und erfindungsgemäß einsetzbar. Es handelt sich dabei um chemisch einander sehr ähnliche Substanzen, die aus cyclischen Polypeptiden aus 11 zum Teil methylierten Aminosäuren bestehen. Cyclosporine sind löslich in Alkohol, Äther, Aceton und chlorierten Kohlenwasserstoffen und natürlichen Ölen (Triglyceriden von Fettsäuren).

Cyclosporin A ist zur oralen Anwendung in Kapseln zum Einnehmen sowie als Lösung zum Einnehmen im Verkehr. Dabei ist bei beiden Darreichungsformen das Cyclosporin gelöst in einer Mischung von Ethanol mit einem Pflanzenöl (Pharmakopoeia Martindale, 29th Edition, US Pharm. XXII, 619 sowie Fachinformation Sandimmun^{R} der Fa. Sandoz).

Neben Ethanol und Pflanzenöl (bevorzugt Maiskeimöl) werden zudem weitere Hilfsstoffe verwendet, um Cyclosporin A in Lösung zu bringen und zu halten, z. B. Poly(oxyethylen)-6-glycerol-tri-(oleat, linolat). Die Verwendung dieser Hilfsstoffe zeigt die große Problematik, bei einer oralen Anwendung Cyclosporin in einer Form zu verabreichen, die eine zumindest teilweise Resorption gewährleistet.

Die immer notwendige Verwendung von Alkohol bedeutet ein gesundheitliches Risiko unter anderem bei Leberkranken, Alkoholkranken, Epileptikern, Hirngeschädigten und Kindern. Außerdem kann durch den Alkohol die Wirkung anderer, gleichzeitig mit Cyclosporin verabreichter Arzneimittel beeinträchtigt oder verstärkt werden.

Auch die zur Erzielung einer besseren Löslichkeit der Cyclosporine verwendeten Poly(oxyethylen)-6-glycerol-tri-oleate oder -linolate können unerwünschte Wirkungen haben, da sie nicht nur die Resorption von Cyclosporin, sondern auch von anderen Stoffen, z. B. Fetten, Paraffinen, Vitaminen etc. beeinflussen. Zudem sollten die verwendeten Mengen an diesen Hilfsstoffen 25 mg pro kg Körpergewicht nicht überschreiten. Außerdem können diese Substanzen unerwünschte allergische Reaktionen, die bis hin zu Schockreaktionen gehen können, auslösen.

Theoretisch wäre es auch möglich, Cyclosporin in reinem Pflanzenöl zu lösen und dann oral zu verabreichen. Praktisch ist dies nicht durchführbar, weil reines Öl vom Patienten nur widerwillig genommen wird und die Resorption unsicher ist.

Das größte Problem der oben beschriebenen Applikationsformen von Cyclosporin(en) zur oralen Anwendung ist jedoch die schlechte und zudem noch stark schwankende Resorption von Cyclosporin. Die Resorption ist unvollständig und variiert stark von Patient zu Patient und sogar beim gleichen Patienten von Tag zu Tag, sie beträgt im allgemeinen zwischen 20 und 50 % der verabreichten Dosis von Cyclosporin(en). Eine gleichmäßige und einheitliche Resorption ist jedoch äußerst wünschenswert. Zudem wäre es sinnvoll, Cyclosporin-Zubereitungen zur Verfügung zu haben, die insgesamt eine bessere Resorption, d. h. eine höhere Bioverfügbarkeit gewährleisten.

Obwohl die hier beschriebenen Probleme seit langem bekannt sind, ist es bisher nicht gelungen, für Cyclosporin(e) eine optimale orale Darreichungsform zu finden.

Eine Aufgabe der vorliegenden Erfindung ist es daher, eine Cyclosporin(e) enthaltende pharmazeutische Zubereitung bereitzustellen, die eine bessere (höhere) und eine gleichmäßigere Resorption von Cyclosporin(en) bei oraler Anwendung ermöglicht. Intra- und interindividuelle Blutspiegelschwankungen von Cyclosporin werden vermindert.

Eine weitere Aufgabe der vorliegenden Erfindung ist es, eine pharmazeutische Zubereitung für Cyclosporin(e) zur Anwendung zur Verfügung zu stellen, die keinen Alkohol enthält.

Eine weitere Aufgabe der vorliegenden Erfindung ist, auf zusätzliche Hilfsstoffe wie z. B. Poly(oxyethylen)-40-Rizinusöl zu verzichten.

Diese Aufgabe(n) wird (werden) erfindungsgemäß dadurch gelöst, daß eine Cyclosporin(e) enthaltende pharmazeutische Zubereitung zur Verfügung gestellt wird, die ein oder mehrere Cyclosporin(e), ein oder mehrere natürliche Öle, 3-sn-Phosphatidylcholin und/oder Phosphatidylethanolamin und Wasser in einer Öl-in-Wasser-Emulsion enthält, wobei (die) Cyclosporin(e) in der öligen Phase enthalten sind.

Die vorliegende Erfindung löst somit überraschenderweise das Problem der relativ schlechten und/oder von Patient zu Patient stark schwankenden Resorption von Cyclosporin(en), indem eine Cyclosporin-haltige orale Applikationsform hergestellt wird, in welcher das Cyclosporin in therapeutisch ausreichender Konzentration in einer Öl-in-Wasser-Emulsion, bestehend aus natürlichen Triglyceriden, 3-sn-Phosphatidylcholinen und Wasser enthalten ist.

Die bisher außerdem verwendeten, evtl. unerwünschten Hilfsstoffe, wie Ethanol können in der erfindungsgemäßen Zubereitung enthalten sein, sind jedoch nicht unbedingt erforderlich.

Mit der erfindungsgemäßen Zubereitung zur oralen Applikation erhält man erfindungsgemäß völlig überraschend eine bessere und zudem gleichmäßigere, d. h. exakter dosierbare Wirksamkeit. Damit kann die Dosis an Cyclosporin(en) vermindert werden und dementsprechend Häufigkeit und Schwere der Nebenwirkungen vermindert werden. Zudem kann, falls dies notwendig erscheint, auf die zusätzliche Anwendung von Ethanol verzichtet werden.

Die höhere und gleichmäßigere Resorption von Cyclosporin(en) wird möglicherweise dadurch erreicht, daß das Cyclosporin in der erfindungsgemäßen Darreichungsform in der Mikrosphäre eines Fettpartikels gelöst ist und dieses Fettpartikel mit dem darin enthaltenen Cyclosporin im Magen-Darm-Trakt in einer Weise gespalten und verstoffwechselt wird, wie dies auch mit natürlichen Nahrungsmitteln, z. B. Milch, geschieht.

Außerdem ist es mit der erfindungsgemäßen pharmazeutischen Zubereitung möglich, die Geschwindigkeit der Resorption des(r) Cyclosporins(e) zu steuern. Die Fettpartikel der Emulsion, in der das Cyclosporin enthalten ist, können nämlich durch Variation der Zusammensetzung des Phosphatidylcholins so hergestellt werden, daß die Freisetzung des Cyclosporins schnell oder langsam erfolgt. So ist es beispielsweise möglich, die pharmazeutische Zubereitung so zusammenzusetzen, daß die Fettpartikel ganz oder teilweise im Magen zerfallen und das(ie) Cyclosporin(e) bereits im Magen freisetzen, oder in einer geänderten Weise, so daß die Freisetzung weitaus langsamer erst im Dünndarm erfolgt und die pharmazeutische Zubereitung den Magen unverändert passiert. Damit erhält man im Prinzip eine magensaftresistente Form einer pharmazeutischen Zubereitung.

Damit erweitern sich die Anwendungsmöglichkeiten erheblich, da man damit Cyclosporin(e) auch erst im Darm freisetzen kann und dadurch eine den physiologischen Verdauungsvorgängen analoge Darreichungsform erhält.

Die erfindungsgemäße pharmazeutische Zubereitung zur oralen Applikation enthält eine therapeutische Menge eines Cyclosporins oder mehrere Cyclosporine, ein pharmazeutisch verträgliches Öl, 3-sn-Phosphatidylcholin und gegebenenfalls ein Alkalisalz einer freien Fettsäure.

Außerdem können Substanzen zur Isotonisierung wie z. B. Glycerin und/oder Sorbit und/oder Xylit enthalten sein.

Bei den erfindungsgemäßen pharmazeutischen Zubereitungen handelt es sich um Öl-in-Wasser-Emulsionen, bei denen die Tröpfchengröße der einzelnen Fett-Tröpfchen unter 10 µm, bevorzugt unter 5 µm, liegt und in der Partikelgröße in etwa der natürlicher Emulsionen wie z. B. Milch entsprechen.

Besonders bevorzugt sind Fettpartikel von mittlerer Größe zwischen 0,05 und 2 µm, ganz besonders mit einem Durchmesser zwischen 0,1 und 1 µm.

Als Öle werden natürliche Öle wie z. B. Sojaöl und/oder Maiskeimöl und/oder Distelöl und/oder Weizenkeimöl und/oder Olivenöl und/oder Sonnenblumenöl und/oder Fischöle und/oder MCT-Öle (mittelkettige Triglyceride = Kokosöl) oder hydrierte oder teilhydrierte natürliche Öle verwendet; die Phosphatidylcholine werden in Form von Sojalecithin oder Eilecithin eingesetzt. Die verwendeten Lecithine können auch teilweise hydriert sein.

Da Cyclosporine in natürlichen Ölen löslich sind, könnte man es als naheliegend betrachten, sie einfach in solchen Ölen zu lösen und zu verabreichen. Dies ist jedoch nicht sinnvoll, da die Patienten das Schlucken von reinem Fett als Öl verweigern. Außerdem entsteht so eine weitaus schlechtere Resorption als bei einer Zubereitung, in der das Öl als eine Öl-in-Wasser-Emulsion vorliegt.

Eine natürlich vorkommende Öl-in-Wasser-Emulsion ist beispielsweise Milch. Durch Zugabe von kristallisiertem (oder auch in beispielsweise Ethanol gelöstem) Cyclosporin gelingt es jedoch nicht, Cyclosporin(e) darin zu lösen, da selbst nach intensivem Rühren der weitaus überwiegende Teil des Cyclosporins immer noch in Form ungelöster Kristalle vorliegt. Dies dürfte darauf zurückzuführen sein, daß das Cyclosporin die Lipid-Hülle nicht durchdringen kann, d. h. ein "Beladen" der Fetttröpfchen einer solchen Emulsion nicht möglich ist. Daher erhält man bei einem solchen Vorgehen eine völlig ungenügende, nicht ausreichende Konzentration des Cyclosporins in der Zubereitung.

Es wurde nun überraschenderweise erfindungsgemäß gefunden, daß es sehr wohl möglich ist, eine oral anwendbare Applikationsform von Cyclosporin(en) ohne Alkohol und ohne Poly(oxyethylen)Derivate in Form einer Emulsion von Cyclosporinen in natürlichen, phamazeutisch verträglichen Ölen, 3-sn-Phosphatidylcholin und Wasser herzustellen. Dabei wird das(ie) verwendete(n) Cyclosporin(e) zunächst in dem verwendeten Öl oder Ölgemisch gelöst, anschließend unter Verwendung von 3-sn-Phosphatidylcholin, bevorzugt in Form von Sojalecithin und/oder Eilecithin, emulgiert und das Gemisch zu einer stabilen Emulsion mit einer Partikelgröße von weniger als 10 µm homogenisiert, bevorzugt im Mittel zwischen 0,05 - 2 µm, insbesondere bevorzugt zwischen 0,1 und 1 µm.

Aus der EP-A-0 391 369 sind Öl-in-Wasser-Emulsionen bekannt, die unter anderem Cyclosporine enthalten können. Diese pharmazeutische Zubereitung ist dadurch gekennzeichnet, daß sie etwa 3-50 % eines Trägeröls enthält, bestehend aus einem MCT-Öl, wahlweise in Kombination mit einem Pflanzenöl, etwa 0,05-20 % eines Phospholipids, etwa 0,03-10 % eines nicht-ionischen Tensids und etwa 0,05-5 % eines ionischen Tensids. Die Verwendung ionischer und nicht-ionischer Tenside ist ein obligatorischer Bestandteil der pharmazeutischen Zusammensetzung der EP-A-0 391 369.

Derartige Tenside (Detergenzien) sind jedoch in der erfindunggemäßen pharmazeutischen Zubereitung unbedingt zu vermeiden. Aufgrund ihrer toxischen Wirkungen sind sie kontraindiziert! Dies gilt sowohl für orale als auch parenterale erfindungsgemäße Zubereitungen.

Die US 5 206 219 offenbart pharmazeutische Zubereitungen zur oralen Applikation, die ein hydrophiles oder ein hydrophobes Tensid enthalten. Derartige Tenside sind jedoch, wie oben ausgeführt, erfindungsgemäß kontraindiziert. Weiterhin enthält die pharmazeutische Zubereitung der US 5 206 219 Propylenglykol und/oder Ethylenglykol in einer Menge von 30 - 60 %. Diese sind intravenös wegen schwerer Nebenwirkungen absolut kontraindiziert. Außerdem enthält diese pharmazeutische Zubereitung keine natürlichen Öle, sondern freie Fettsäuren in Mengen von 30 - 60 %. Freie Fettsäuren, z.B. Ölsäure, können in derart hohen Mengen intravenös nicht verabreicht werden, da hier die Gefahr einer Hämolyse des Blutes besteht.

Erfindungsgemäß sind demnach oral oder parenteral ionische und nicht-ionische Detergenzien zu vermeiden. Weiterhin sind Polyole wie Propylenglykol und Polyethylenglykol zu vermeiden. Ein besonderer Vorteil der erfindungsgemäßen pharmazeutischen Zubereitung zur oralen Applikation liegt darin, daß bei Verwendung von Sojalecithin die Beimengung eines Alkalisalzes einer freien Fettsäure nicht notwendig ist.

Ist eine sterile Emulsion zur oralen Anwendung erwünscht, kann eine Sterilisation durch Hitze erfolgen, bzw. eine Sterilisation oder Konservierung durch entsprechende Filtration und/oder Konservierungsmittel.

Die enthaltene pharmazeutische Zubereitung zur oralen Anwendung von Cyclosporin(en) enthält keine bedenklichen Hilfsstoffe und ermöglicht eine bessere und gleichmäßigere Resorption.

Als Cyclosporine können natürliche und synthetische Cyclosporine, beispielsweise die bekannten Cyclosporine A - Z verwendet werden; bevorzugt sind Cyclosporin A und Cyclosporin G sowie das Cyclosporin-Derivat SDZ IMM 125.

Als natürliche Öle können verwendet werden z. B. Sojaöl, Distelöl (Safloröl), Kokosöle (MCT-Öle), FIschöle, Maiskeimöl, Weizenkeimöl, Olivenöl, Sonnenblumenöl, Baumwollöl, Walnußöl etc. Die Öle können auch teilweise hydriert sein.

Selbstverständlich sollte das Öl möglichst arm an Peroxiden sein und arm an Sauerstoff, um nicht ranzig zu werden. Antioxidationsmittel, wie z. B. Vitamin E, oder andere Antioxidantien können enthalten sein.

Das verwendete Cyclosporin, z. B. Cyclosporin A, wird zunächst in dem Öl, z. B. Maiskeimöl oder Sojaöl gelöst, evtl. in der Wärme. Dabei werden im allgemeinen Konzentrationen zwischen 1 - 10 Gewichtsprozent erreicht.

Es sollte darauf geachtet werden, daß dabei das Vorhandensein von Sauerstoff möglichst ausgeschlossen wird, um eine Oxidation des Öls zu vermeiden.

Zu der Cyclosporin-haltigen, öligen Lösung wird nun Wasser, gegebenenfalls keinen Sauerstoff enthaltend, gegeben sowie als Emulgator 3-sn-Phosphatidylcholin, bevorzugt in Form von Eilecithin oder Sojalecithin. Auf 100 Teile Öl werden im allgemeinen 4 - 20 Teile des 3-sn-Phosphatidylcholins gegeben. Das 3-sn-Phosphatidylcholin kann auch teilweise hydriert oder hydriert sein.

Bevorzugt sind Eilecithin mit einem Gehalt von 3-sn-Phosphatidylcholin von 50 - 85 % sowie Sojalecithin mit einem Gehalt an 3-sn-Phosphatidylcholin von 20 - 80 %.

Als Lieferanten des 3-sn-Phosphatidylcholins, des teilhydrierten 3-sn-Phosphatidylcholins oder hydrierten 3-sn-Phosphatidylcholins können beispielweise pflanzliche Lecithine aus Soja oder Raps, Lecithin aus Ei und/oder Lecithin aus Hirn dienen. Bevorzugt sind Sojalecithin und/oder Eilecithin.

Gegebenenfalls kann außerdem ein Alkalisalz einer freien Fettsäure mit 6 - 26 Kohlenstoffatomen zugegeben werden, um den pH-Wert auf ca. 5 - 9 einzustellen und die Emulgierung und spätere Homogenisierung zu erleichtern. Bevorzugt sind die Natrium- und Kaliumsalze der Palmitinsäure, Palmitoleinsäure, Stearinsäure, Ölsäure, Linolsäure und Linolensäure. Auch Gemische dieser Salze können eingesetzt werden.

Das Gemisch, welches nun Cyclosporin(e), natürliches Öl (evtl. teilhydriert oder hydriert), 3-sn-Phosphatidylcholin, Wasser und gegebenenfalls ein oder mehrere Alkalisalze freier Fettsäuren enthält, wird nun mit Wasser soweit verdünnt, daß das Öl 5 - 80 % des Gesamtgewichts, bevorzugt 20 - 50 % des Gesamtgewichts beträgt.

Es wird nun durch starkes Rühren, bevorzugt mit einem Ultra-Turrax, eine Rohemulsion hergestellt.

Diese Rohemulsion wird nun mit einem Hochdruck-Homogenisator bei Drücken zwischen 100 - 500 bar so lange, unter Umständen mehrmals, homogenisiert, bis man eine Emulsion erhält, bei der die Partikelgröße aller Partikel unter 20 µm, bevorzugt unter 10 µm, besonders bevorzugt unter 5 µm liegt. Dabei haben im Mittel die Fettpartikel eine Größe von 0,05- 10 µm, bevorzugt zwischen 0,05 und 2 µm, besonders bevorzugt zwischen 0,1 und 1 µm. Der verwendete Hochdruck-Homogenisator sollte vorzugsweise drei Kolben besitzen.

Danach wird die Emulsion gegebenenfalls durch Zugabe von Wasser auf die gewünschte Konzentration verdünnt. Anschließend kann die Emulsion in geeignete Darreichungsformen, z. B. in Trinkfläschchen und/oder Trinkampullen oder auch in Weichgelatine-Kapseln abgefüllt werden.

Um die orale Darreichungsform vor der Zersetzung durch Bakterien zu schützen, kann gegebenenfalls eine Hitzesterilisation durchgeführt werden und/oder die Darreichungsform wird durch den Zusatz üblicher Konservierungsmittel wie z. B. Benzoesäure, Sorbinsäure, p-Hydroxybenzoesäureester etc. geschützt.

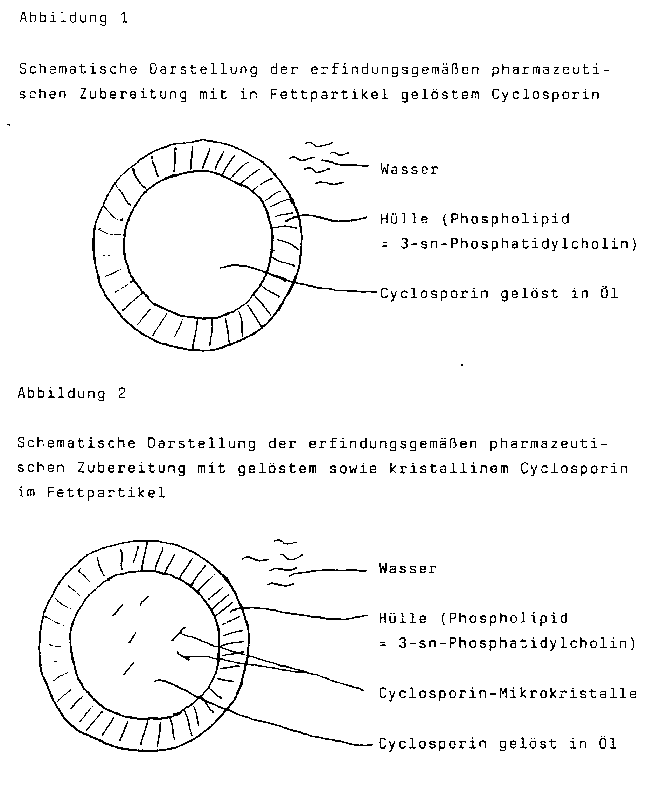

Üblicherweise und vorzugsweise liegt das in der oralen Applikationsform enthaltene Cyclosporin in vollständig gelöster Form vor. Dabei ist das Cyclosporin, gelöst im Öl, im Inneren eines Fettpartikels enthalten, wobei das Öl mit einer Hülle, bestehend im wesentlichen aus Phospholipid (3-sn-Phosphatidylcholin), umgeben ist. Die beiliegende Abbildung 1 zeigt in schematischer Form den Aufbau der erfindungsgemäßen, Cyclosporin enthaltenden Fettpartikel.

Es ist jedoch sogar auch möglich, das Cyclosporin bei der Herstellung in so hoher Konzentration anzuwenden, daß in der schließlich erhaltenen endgültigen oralen Applikationsform nur ein Teil des Cyclosporins gelöst vorliegt, ein anderer Teil sich jedoch in fester Form im Inneren des Fettpartikels befindet. Dies geschieht in der Weise, daß bei erhöhter Temperatur (z. B. 50 - 80°C) das Cyclosporin im Öl gelöst wird und damit eine bei Raumtemperatur an Cyclosporin übersättigte Lösung erhalten wird. Anschließend wird bei erhöhter Temperatur emulgiert und homogenisiert. Nach Herstellen der Emulsion erfolgt eine Abkühlung auf Raumtemperatur, wobei ein Teil des sich im Inneren der Fettpartikel enthaltenen Cyclosporins auskristallisiert. Wegen der das Fettpartikel umgebenden Hülle, welche das feste Cyclosporin nicht durchdringen kann, bleibt das Cyclosporin eingeschlossen.

Die beiliegende Abbildung 2 zeigt in schematischer Form ein solches Fettpartikel.

Die Emulsion kann in Form von Trinkampullen, Tropfen, Weichgelatine-Kapseln oral verabreicht werden. Im Magen-Darm-Trakt werden die Fettpartikel mit dem darin eingeschlosssenen Cyclosporin durch die dort enthaltenen Enzyme gespalten und damit das Cyclosporin freigesetzt. Da die Fettpartikel, vergleichbar der natürlichen Fettemulsion Milch, im Magen nur wenig angegriffen werden, erfolgt die Freisetzung vor allem im Dünndarm, wo die Fettpartikel in natürlicher Weise durch das dort enthaltene Pankreatin aufgespalten werden und das Cyclosporin freisetzen. Dadurch wird eine bessere Resorption erzielt und auch eine gleichmäßigere und sicherere Aufnahme in den Organismus.

Die folgenden Beispiele beschreiben die erfindungsgemäße pharmazeutische Zubereitung und das Verfahren zu ihrer Herstellung im einzelnen. Die Erfindung ist nicht darauf beschränkt.

### Beispiel 1

10 kg winterisiertes Sojaöl (neutraler pH-Wert, peroxidfrei) wird mit Stickstoff begast und auf ca. 50°C erwärmt. (Unter winterisiertem Sojaöl wird Sojaöl verstanden, das vor der Anwendung auf unter -10°C abgekühlt wird, damit in der Kälte unlösliche Teile ausfallen, die abfiltriert werden.) Zu diesem Sojaöl werden nun 400 g Cyclosporin A gegeben und unter Rühren gelöst.

In ein zweites Gefäß werden 13,8 kg Wasser gegeben und mit Stickstoff begast. Dann werden 1,2 kg Eilecithin mit einem Gehalt von ca. 70 % Phosphatidylcholin (und ca. 18 % Phosphatidylethanolamin) zugegeben sowie 50 g Natriumoleat. Durch starkes Rühren mit einem Ultra-Turrax wird bei einer Temperatur von ca. 50°C eine rohe Emulsion hergestellt.

Nun wird das Sojaöl mit dem darin gelösten Cyclosporin A zugegeben und ca. 5 Minuten mit dem Ultra-Turrax weiter gerührt.

Die so erhaltene Rohemulsion wird in einem Hochdruckhomogenisator bei 100 - 500 bar homogenisiert. Der Vorgang der Homogenisierung wird so oft wiederholt, bis die gewünschte Größe der Fettpartikel erreicht ist. Bei jedem Homogenisierungsvorgang verkleinert sich die mittlere Tröpfchengröße. Nach 4maligem Homogenisieren erhält man eine Tröpfchengröße von im Mittel 0,2 - 0,6 µm.

Nach jedem Homogenisierungsvorgang sollte die Emulsion auf ca. 30 - 50 °C abgekühlt werden.

Die Größenverteilung der das Cyclosporin enthaltenden Fettpartikel ist wie folgt:

| Partikelgröße | Zahl der Teilchen |
|---|---|
| < 0,2 µm | 15 % |
| 0,2 - 0,5 µm | 56 % |
| 0,5 - 0,9 µm | 19 % |
| 0,9 - 1,2 µm | 6 % |
| 1,2 - 1,5 µm | 2 % |
| 1,5 - 1,9 µm | unter 1 % |
| 1,9 - 2,2 µm | unter 1 % |
| 2,2 - 3,2 µm | unter 1 % |
| > 3,2 µm | 0 % |

Man erhält ca. 25 kg einer Emulsion, die insgesamt 400 g Cyclosporin A enthält. D. h. ca. 100 mg Cyclosporin sind in 6 ml der Emulsion enthalten.

Damit die Emulsion bei der Aufnahme einen guten Geschmack aufweist, können Aromastoffe zugegeben werden.

Die Emulsion wird in Trinkampullen zu 10 ml abgefüllt und in üblicher Weise hitzesterilisiert.

### Beispiel 2

Das Verfahren des Beispiels 1 wird wiederholt, wobei statt des Sojaöls ein Fischölkonzentrat, das 18 % Eicosapentaensäure und 12 % Docosahexaensäure enthält, verwendet wird.

### Beispiel 3

Das Beispiel 1 wird wiederholt, wobei statt des in Beispiel 1 verwendeten Eilecithins ein Sojalecithin mit einem Gehalt an 3-sn-Phosphatidylcholin von 60 % verwendet wird.

### Beispiel 4

Das Beispiel 1 wird wiederholt, wobei anstelle des Eilecithins ein teilhydriertes Eilecithin mit ca. 80 % teilhydriertem 3-sn-Phosphatidylcholin eingesetzt wird.

### Beispiel 5

10 kg Sojaöl mit einem Gehalt an freien Fettsäuren von 2,5 meq/l und einem pH-Wert von 3,5 - 4,5 wird auf ca. 50°C erwärmt und es werden 500 g Cyclosporin A zugegeben. Entsprechend Beispiel 1 wird eine Rohemulsion hergestellt durch Rühren mit einem Ultra-Turrax. Anschließend wird durch Zugabe einer 10%igen Natriumhydroxidlösung ein pH-Wert von 6,5 - 8,5 eingestellt. Die weitere Herstellung erfolgt entsprechend Beispiel 1.

### Beispiel 6

Das Beispiel 2 wird wiederholt, wobei jedoch statt 400 g Cyclosporin A 250 g Cyclosporin A und 250 g Cyclosporin G eingesetzt werden.

### Beispiel 7

Eine Cyclosporin enthaltende pharmazeutische Zubereitung wird, wie im Beispiel 1 beschrieben, hergestellt, wobei jedoch statt des Cyclosporin A 150 g von SDZ IMM 125, ein Hydroxyethylderivat des D-Serin-8-Cyclosporins, eingesetzt wird. Die Herstellung und die Struktur von SDZ IMM 125 sind beschrieben in G. Baumann et al., Transplantation Proceedings Vol. 24, No. 4, Suppl. 2, Seite 31 - 38 (1992).

### Beispiel 8

Dieses Beispiel zeigt die Freisetzung des Cyclosporins aus der erfindungsgemäßen pharmazeutischen Zubereitung in vitro.
a) 10 ml der pharmazeutischen Zubereitung nach dem Beispiel 1 werden in 50 ml künstlichem Magensaft nach USP XXII enthaltend Salzsäure, Natriumchlorid und Pepsin gegeben mit einem pH-Wert von 1,2 und 2 Stunden bei 37°C gerührt. Die Emulsion bleibt praktisch unverändert.
b) 10 ml der pharmazeutischen Zubereitung nach dem Beispiel 1 werden in 50 ml künstlichem Darmsaft nach USP XXII enthaltend Pankreatin mit einem pH-Wert von 7,5 gegeben und 1 Stunde bei 37°C gerührt. Die Emulsion bleibt nicht stabil, sondern die Fettpartikel zerfallen und setzen das Cyclosporin aus den Fettpartikeln frei.

Bei der parenteralen Applikation wird die nachfolgende pharmazeutische Zusammensetzung bevorzugt eingesetzt:

| | | pro Liter |
|---|---|---|
| 1. | Sojaöl | bis 400 g |
| 2. | Eilecithin | 5- 20 g |
| | dabei muß das Eilecithin zwischen 60 % und 85 % Phosphatidylcholin enthalten | |
| 3. | Natriumsalz oder Kaliumsalz einer Fettsäure mit mindestens 10 Kohlenstoffatomen: 0,2 - 1,0 g. Statt des Salzes kann auch eine Alkalilauge + Fettsäure verwendet werden. | |

In der erfindungsgemäßen pharmazeutischen Zubereitung zur oralen Applikation liegt das teilweise hydrierte 3-sn-Phosphatidylcholin bevorzugt in der Form von teilweise hydriertem Sojalecithin oder teilweise hydriertem Eilecithin vor, wobei vorzugsweise das Eilecithin mindestens 60 Gew.% 3-sn-Phosphatidylcholin, weiterhin bevorzugt zwischen 60 - 85 Gew.% enthält.

Der Gehalt an 3-sn-Phosphatidylcholinen in der erfindungsgemäßen pharmazeutischen Zubereitung liegt bevorzugt bei 0,3 - 4, weiterhin bevorzugt bei 0,6 - 2,4 Gew.%. Der Gehalt der 3-sn-Phosphatidylcholin enthaltenden Substanzen in der erfindungsgemäßen pharmazeutischen Zubereitung liegt bevorzugt bei 0,5 - 6,5, weiterhin bevorzugt bei 1 - 4 Gew.%.

Vorteilhafterweise liegt in der erfindungsgemäßen Zusammensetzung die Konzentration der Cyclosporine im Öl zwischen 0,2 - 10 Gew.%, bevorzugt zwischen 1 - 5 Gew.%.

Der Gehalt an freien Fettsäuren und/oder deren Alkalisalzen liegt bevorzugt zwischen 0,1 Gew.% und 3 Gew.%, weiterhin bevorzugt zwischen 0,5 Gew.% und 1,5 Gew.%.

Zur Herstellung einer Cyclosporin enthaltenden pharmazeutischen Zubereitung, die ein Gemisch aus Cyclosporin(en), Ölen natürlicher Herkunft, 3-sn-Phosphatidylcholin und Wasser und bereits 0,01 bis 1 % an freien natürlichen Fettsäuren und/oder deren Alkalisalzen enthält, wird dieses Gemisch mittels Alkalilauge(n) vor der Emulgierung auf einen pH-Wert von 5 - 10, bevorzugt 7 - 9, eingestellt.

Bei der Herstellung werden zur Einstellung des pH-Wertes der Emulsion auf einen Wert von 5 - 10, bevorzugt 7 - 9, Fettsäuren und/oder deren Alkalisalze verwendet.

Bei der Herstellung der Cyclosporin(e) enthaltenden pharmazeutischen Zubereitung der vorliegenden Erfindung werden bevorzugt zusätzlich pharmazeutisch verträgliche gesättigte und/oder ungesättigte Fettsäuren und/oder Alkalisalze der Fettsäuren eingesetzt.

## Patentansprüche

1. Cyclosporine enthaltende pharmazeutische Zubereitung zur oralen Applikation,
dadurch gekennzeichnet,
daß sie ein oder mehrere Cyclosporin(e), ein oder mehrere Öle natürlicher Herkunft, 3-sn-Phosphatidylcholin und/oder Phosphatidylethanolamin und Wasser enthält, jedoch Seine nicht-ionische Tenside und/oder Gallenwegstenside, Gallensäure und ihre Derivate.

2. Cyclosporin(e) enthaltende pharmazeutische Zubereitung nach Anspruch 1.
dadurch gekennzeichnet,
daß zusätzlich pharmazeutisch verträgliche freie Fettsäuren und/oder Alkalisalze freier Fettsäuren enthalten sind.

3. Cyclosporin(e) enthaltende pharmazeutische Zubereitung nach Anspruch 1 und 2,
dadurch gekennzeichnet,
daß das Cyclosporin ein natürliches und/oder synthetisches Cyclosporin und/oder ein Cyclosporin-Derivat ist, bevorzugt ein Cyclosporin A und/oder ein Cyclosporin G und/ oder das Cyclosporin-Derivat SDZ IMM 125.

4. Cyclosporin(e) enthaltende pharmazeutische Zubereitung zur oralen Applikation,
dadurch gekennzeichnet,
daß das 3-sn-Phosphatidylcholin in Form von 3-sn-Phosphatidylcholin enthaltenden Substanzen vorliegt, und daß vorzugsweise die 3-sn-Phosphatidylcholin enthaltende Substanz Sojalecithin (Sojaphosphatid) und/oder Eilecithin ist.

5. Cyclosporin(e) enthaltende pharmazeutische Zubereitung zur oralen Applikation nach einem oder mehreren der vorhergehenden Ansprüche,
dadurch gekennzeichnet,
daß das verwendete 3-sn-Phosphatidylcholin teilweise oder vollständig hydriert ist.

6. Cyclosporin(e) enthaltende pharmazeutische Zubereitung zur oralen Applikation nach einem oder mehreren der vorhergehenden Ansprüche,
dadurch gekennzeichnet,
daß als Öle natürlicher Herkunft Sojaöl und/oder Maiskeimöl und/oder Fischöl und/oder Distelöl und/oder Weizenkeimöl und/oder Olivenöl und/oder deren teilhydrierten oder hydrierten Öle und/oder mittelkettige Triglyceride (z. B. Kokosöl) verwendet werden und daß vorzugsweise das Öl frei von Peroxiden ist.

7. Cyclosporin(e) enthaltende pharmazeutische Zubereitung zur oralen Applikation nach einem oder mehreren der vorhergehenden Ansprüche,
dadurch gekennzeichnet,
daß die Konzentration der Cyclosporine im Öl zwischen 0,2 - 10 Gew.%, bevorzugt zwischen 1 - 5 Gew.% liegt.

8. Cyclosporin(e) enthaltende pharmazeutische Zubereitung zur oralen Applikation nach einem oder mehreren der vorhergehenden Ansprüche,
dadurch gekennzeichnet,
daß das natürliche Öl in einem Anteil von 5 - 80 %, bevorzugt in einem Anteil von 20 - 50 % des Gesamtgewichts in der pharmazeutischen Zubereitung vorliegt.

9. Cyclosporin(e) enthaltende pharmazeutische Zubereitung zur oralen Applikation nach einem oder mehreren der vorhergehenden Ansprüche,
dadurch gekennzeichnet,
daß als pharmazeutisch verträgliche Fettsäuren gesättigte und/oder ungesättigte Fettsäuren und/oder deren Alkalisalze, bevorzugt mit 6 - 26 Kohlenstoffatomen, enthalten sind.

10. Cyclosporin(e) enthaltende pharmazeutische Zubereitung zur oralen Applikation nach einem oder mehreren der vorhergehenden Ansprüche,
dadurch gekennzeichnet,
daß der Gehalt an freien Fettsäuren und/oder deren Alkalisalzen zwischen 0,1 Gew.% und 3 Gew.%, bevorzugt zwischen 0,5 Gew.% und 1,5 Gew.%, beträgt.

11. Cyclosporin(e) enthaltende pharmazeutische Zubereitung zur oralen Applikation nach einem oder mehreren der vorhergehenden Ansprüche,
dadurch gekennzeichnet,
daß die in den natürlichen Ölen und/oder hydrierten und/oder teilhydrierten natürlichen Ölen gelösten Cyclosporine im Innern der aus diesen Ölen bestehenden Fettpartikel eingeschlossen, von einer Hülle aus 3-sn-Phosphatidylcholin und/oder Phosphatidylethanolamin umgeben und in Wasser emulgiert sind.

12. Cyclosporin(e) enthaltende pharmazeutische Zubereitung zur oralen Applikation nach einem oder mehreren der vorhergehenden Ansprüche,
dadurch gekennzeichnet,
daß die mittlere Größe der Fettpartikel in der Emulsion zwischen 0,05 und 10 µm, bevorzugt zwischen 0,05 und 2 µm und insbesondere bevorzugt zwischen 0,1 bis 1 µm beträgt.

13. Cyclosporin(e) enthaltende pharmazeutische Zubereitung zur oralen Applikation nach einem oder mehreren der vorhergehenden Ansprüche,
dadurch gekennzeichnet,
daß nach der oralen Verabreichung der Zubereitung ein wesentlicher Anteil des Cyclosporins nicht im Magen, sondern erst im Dünndarm freigesetzt wird.

14. Verfahren zur Herstellung der Cyclosporin(e) enthaltenden pharmazeutischen Zubereitung zur oralen Applikation nach einem oder mehreren der vorhergehenden Ansprüche,
dadurch gekennzeichnet,
daß die Cyclosporine in natürlichen Ölen gelöst und anschließend mit 3-sn-Phosphatidylcholin und/oder Phosphatidylethanolamin und Wasser in an sich bekannter Weise emulgiert werden.

15. Verfahren nach Anspruch 14,
dadurch gekennzeichnet,
daß die Cyclosporine vor der Emulgierung in dem Öl natürlicher Herkunft vollständig gelöst und anschließend mit Hilfe von 3-sn-Phosphatidylcholinen und/oder deren Alkalisalzen in Wasser zu einer Rohemulsion emulgiert werden und das Gemisch anschließend zu einer Emulsion, bevorzugt mit einer Partikelgröße von unter 10 µm, insbesondere bevorzugt unter 2 µm homogenisiert wird.

16. Verfahren zur Herstellung der Cyclosporin(e) enthaltenden pharmazeutischen Zubereitung zur oralen Applikation nach einem oder mehreren der vorhergehenden Ansprüche,
dadurch gekennzeichnet,
daß das Gemisch aus Cyclosporin(en), Ölen natürlicher Herkunft, 3-sn-Phosphatidylcholin und Wasser bereits 0,01 bis 1 % an freien natürlichen Fettsäuren und/oder deren Alkalisalzen enthält und dieses Gemisch mittels Alkalilauge(n)vor der Emulgierung auf einen pH-Wert von 5 - 10, bevorzugt 7 - 9, eingestellt wird.

17. Verfahren zur Herstellung der Cyclosporin(e) enthaltenden pharmazeutischen Zubereitung zur oralen Applikation nach einem oder mehreren der vorhergehenden Ansprüche,
dadurch gekennzeichnet,
daß Fettsäuren und/oder deren Alkalisalze verwendet werden, um die Emulsion auf einen pH-Wert von 5 - 10, bevorzugt 7 - 9, einzustellen.

18. Verfahren zur Herstellung der Cyclosporin(e) enthaltenden pharmazeutischen Zubereitung zur oralen Applikation nach einem oder mehreren der vorhergehenden Ansprüche,
dadurch gekennzeichnet,
daß zur Herstellung der pharmazeutischen Zubereitung im wesentlichen sauerstofffreie Lösungen verwendet werden.

19. Verfahren zur Herstellung der Cyclosporin(e) enthaltenden pharmazeutischen Zubereitung zur oralen Applikation nach einem oder mehreren der vorhergehenden Ansprüche,
dadurch gekennzeichnet,
daß zusätzlich pharmazeutisch verträgliche gesättigte und/oder ungesättigte Fettsäuren und/oder Alkalisalze der Fettsäuren verwendet werden.

20. Verfahren zur Herstellung der Cyclosporin(e) enthaltenden pharmazeutischen Zubereitung zur oralen Applikation nach einem oder mehreren der vorhergehenden Ansprüche,
dadurch gekennzeichnet,
daß durch intensives Rühren zunächst eine Rohemulsion hergestellt wird und anschließend die endgültige Emulgierung in einem Hochdruck-Homogenisator bei Drücken zwischen 100 - 500 bar erfolgt, wobei vorzugsweise der Hochdruck-Homogenisator drei Kolben besitzt.

21. Verwendung der pharmazeutischen Zubereitung nach einem oder mehreren der vorhergehenden Ansprüche zur oralen Applikation.

## Claims

1. A cyclosporins containing pharmaceutical preparation for oral application
characterized in that
it contains one or more cyclosporin(s), one or more oils of natural origin, 3-sn-phosphatidyl choline and/or phosphatidyl ethanol amine and water but no non-ionic surfactants and/or bile duct surfactants, bile acid and bile acid derivatives.

2. A cyclosporin(s) containing pharmaceutical preparation according to claim 1,
characterized in that
it further comprises pharmaceutically tolerable free fatty acids and/or alkali salts of free fatty acids.

3. A cyclosporin(s) containing pharmaceutical preparation according to claims 1 and 2,
characterized in that
said cyclosporin is a natural and/or synthetic cyclosporin and/or cyclosporin derivative, preferably a cyclosporin A and/or a cyclosporin G and/or the cyclosporin derivative SDZ IMM 125.

4. A cyclosporin(s) containing pharmaceutical preparation for oral application,
characterized in that
the 3-sn-phosphatidyl choline is present in the form of substances containing 3-sn-phosphatidyl choline, and that preferably the 3-sn-phosphatidyl choline containing substance is soy bean lecithin (soy bean phosphatide) and/or egg lecithin.

5. A cyclosporin(s) containing pharmaceutical preparation for oral application according to one or more of the preceding claims,
characterized in that
the used 3-sn-phosphatidyl choline is partially or completely hydrogenated.

6. A cyclosporin(s) containing pharmaceutical preparation for oral application according to one or more of the preceding claims,
characterized in that
as oils of natural origin are used soy bean oil and/or corn oil and/or fish oil and/or safflower oil and/or wheat germ oil and/or olive oil and/or partly or completely hydrogenated oils thereof and/or medium-chain triglycerides (e.g. coconut oil), and that preferably the oil is free of peroxides.

7. A cyclosporin(s) containing pharmaceutical preparation for oral application according to one or more of the preceding claims,
characterized in that
the concentration of the cyclosporins in the oil is between 0.2 - 10.0% by weight, preferably between 1 - 5% by weight.

8. A cyclosporin(s) containing pharmaceutical preparation for oral application according to one or more of the preceding claims,
characterized in that
the natural oil amounts to 5 - 80%, preferably 20 - 50% of the total weight of the pharmaceutical preparation.

9. A cyclosporin(s) containing pharmaceutical preparation for oral application according to one or more of the preceding claims,
characterized in that
as pharmaceutically tolerable free fatty acids saturated and/or unsaturated fatty acids and/or alkali salts thereof are comprised, preferably having 6-26 carbon atoms.

10. A cyclosporin(s) containing pharmaceutical preparation for oral application according to one or more of the preceding claims,
characterized in that
the concentration of free fatty acids and/or the alkali salts thereof lies within the range of 0.1 to 3.0% by weight, preferably 0.5 to 1.5% by weight.

11. A cyclosporin(s) containing pharmaceutical preparation for oral application according to one or more of the preceding claims,
characterized in that
the cyclosporins which are dissolved in natural oils and/or hydrogenated and/or partly hydrogenated natural oils are enclosed in the interior of fatty particles consisting of said oils, and are surrounded by an envelope of 3-sn-phosphatidyl choline and/or phosphatidyl ethanol amine and are being emulsified in water.

12. A cyclosporin(s) containing pharmaceutical preparation for oral application according to one or more of the preceding claims,
characterized in that
the mean size of said fatty particles in said emulsion is between 0.05 and 10µm, preferably between 0.05 and 2µm and especially preferably between 0.1 and 1µm.

13. A cyclosporin(s) containing pharmaceutical preparation for oral application according to one or more of the preceding claims,
characterized in that
following the oral administration of said preparation a substantial portion of the cyclosporin is not released in the stomach, but only in the small intestines.

14. A process for the production of the cyclosporin(s) containing pharmaceutical preparation for oral application according to one or more of the preceding claims, characterized in that
said cyclosporins are dissolved in natural oils; and are subsequently emulsified with 3-sn-phosphatidyl choline and/or phosphatidyl ethanol amine in a manner known per se.

15. A process according to claim 14
characterized in that
the cyclosporins are dissolved completely in said oil of natural origin prior to emulsion and are subsequently emulsified in water to a crude emulsion by means of 3-sn-phosphatidyl cholines and/or the alkali salts thereof, and that subsequently said mixture is homogenized to an emulsion, preferably with a particle size of less than 10µm, especially preferably less than 2µm.

16. A process for the production of the cyclosporin(s) containing pharmaceutical preparation for oral application according to one or more of the preceding claims, characterized in that
said mixture of cyclosporin(s), oils of natural origin, 3-sn-phosphatidyl choline and water already contains 0.01 to 1% of free fatty acids and/or the alkali salts thereof, and the pH value of said mixture is adjusted to 5 - 10, preferably 7 - 9, by means of alkali lye(s).

17. A process for the production of the cyclosporin(s) containing pharmaceutical preparation for oral application according to one or more of the preceding claims, characterized in that
said fatty acids and all their alkali salts are used to adjust the pH value of the emulsion to 5 - 10, preferably 7-9.

18. A process for the production of the cyclosporin(s) containing pharmaceutical preparation for oral application according to one or more of the preceding claims, characterized in that
for the production of said pharmaceutical preparation substantially oxygen-free solutions are used.

19. A process for the production of the cyclosporin(s) containing pharmaceutical preparation for oral application according to one or more of the preceding claims, characterized in that
additionally pharmaceutically tolerable saturated and/or unsaturated fatty acids and/or the alkali salts of said fatty acids are used.

20. A process for the production of the cyclosporin(s) containing pharmaceutical preparation for oral application according to one or more of the preceding claims, characterized in that
first a crude emulsion is produced by intense stirring and subsequently the final emulsification takes place in a high pressure homogenizer at pressures of between 100-500 bar, said high pressure homogenizer preferably having three pistons.

21. Use of the pharmaceutical preparation according to one or more of the preceding claims for oral administration.

## Revendications

1. Composition pharmaceutique pour administration orale, contenant des cyclosporines, caractérisée en ce qu'elle contient une ou plusieurs cyclosporine(s), une ou plusieurs huiles d'origine naturelle, de la 3-sn-phosphatidylcholine et/ou de la phosphatidyléthanolamine et de l'eau, mais ne contient pas de tensioactifs non ioniques et/ou de tensioactifs de la voie billiaire, d'acide cholique et leur derivés.

2. Composition pharmaceutique contenant une(des) cyclosporine(s), selon la revendication 1, caractérisée en ce que sont en outre contenus des acides gras libres et/ou des sels alcalins d'acides gras libres, pharmaceutiquement acceptables.

3. Composition pharmaceutique contenant une(des) cyclosporine(s), selon la revendication 1 ou 2, caractérisée en ce que la cyclosporine est une cyclosporine naturelle et/ou synthétique et/ou un dérivé de cyclosporine, de préférence une cyclosporine A et/ou une cyclosporine G et/ou le dérivé de cyclosporine SDZ IMM 125.

4. Composition pharmaceutique contenant une(des) cyclosporine(s), pour administration orale, caractérisée en ce que la 3-sn-phosphatidylcholine se trouve sous forme de substances contenant de la 3-sn-phosphatidylcholine, et en ce que la substance contenant de la 3-sn-phosphatidylcholine est de préférence la lécithine de soja (phosphatide de soja) et/ou la lécithine de jaune d'oeuf.

5. Composition pharmaceutique contenant une(des) cyclosporine(s), pour administration orale, selon une ou plusieurs des revendications précédentes, caractérisée en ce que la 3-sn-phosphatidylcholine utilisée est partiellement ou totalement hydrogénée.

6. Composition pharmaceutique contenant une(des) cyclosporine(s), pour administration orale, selon une ou plusieurs des revendications précédentes, caractérisée en ce que l'on utilise comme huiles d'origine naturelle l'huile de soja et/ou l'huile de germes de maïs et/ou l'huile de poisson et/ou l'huile de chardon et/ou l'huile de germes de blé et/ou l'huile d'olive, et/ou leurs huiles hydrogénées ou partiellement hydrogénées et/ou des triglycérides à longueur de chaîne moyenne (par exemple l'huile de coprah), et en ce que l'huile est de préférence exempte de peroxydes.

7. Composition pharmaceutique contenant une(des) cyclosporine(s), pour administration orale, selon une ou plusieurs des revendications précédentes, caractérisée en ce que la concentration des cyclosporines dans l'huile est comprise entre 0,2 et 10 % en poids, de préférence entre 1 et 5 % en poids.

8. Composition pharmaceutique contenant une(des) cyclosporine(s), pour administration orale, selon une ou plusieurs des revendications précédentes, caractérisée en ce que l'huile naturelle est présente dans la composition pharmaceutique en une proportion de 5 - 80 %, de préférence en une proportion de 20 - 50 % du poids total.

9. Composition pharmaceutique contenant une(des) cyclosporine(s), pour administration orale, selon une ou plusieurs des revendications précédentes, caractérisée en ce que sont contenus, en tant qu'acides gras pharmaceutiquement acceptables, des acides gras saturés et/ou non saturés et/ou leurs sels alcalins, ayant de préférence de 6 à 26 atomes de carbone.

10. Composition pharmaceutique contenant une(des) cyclosporine(s), pour administration orale, selon une ou plusieurs des revendications précédentes, caractérisée en ce que la teneur en acides gras libres et/ou en leurs sels alcalins est comprise entre 0,1 % en poids et 3 % en poids, de préférence entre 0,5 % en poids et 1,5 % en poids.

11. Composition pharmaceutique contenant une(des) cyclosporine(s), pour administration orale, selon une ou plusieurs des revendications précédentes, caractérisée en ce que les cyclosporines dissoutes dans les huiles naturelles et/ou dans les huiles naturelles hydrogénées et/ou partiellement hydrogénées sont incluses à l'intérieur des particules lipidiques constituées de ces huiles, entourées d'une enveloppe de 3-sn-phosphatidylcholine et/ou de phosphatidyléthanolamine et émulsionnées dans l'eau.

12. Composition pharmaceutique contenant une (des) cyclosporine(s), pour administration orale, selon une ou plusieurs des revendications précédentes, caractérisée en ce que la taille moyenne des particules lipidiques dans l'émulsion est comprise entre 0,05 et 10 µm, de préférence entre 0,05 et 2 µm et, de façon particulièrement préférée, entre 0,1 et 1 µm.

13. Composition pharmaceutique contenant une(des) cyclosporine(s), pour administration orale, selon une ou plusieurs des revendications précédentes, caractérisée en ce que, après l'administration orale de la composition, une proportion substantielle de la cyclosporine n'est pas libérée dans l'estomac, mais l'est seulement dans l'intestin grêle.

14. Procédé pour la préparation de la composition pharmaceutique contenant une (des) cyclosporine(s), pour administration orale, selon une ou plusieurs des revendications précédentes, caractérisé en ce que les cyclosporines sont dissoutes dans des huiles naturelles et ensuite émulsionnées, d'une façon connue en soi, avec de la 3-sn-phosphatidylcholine et/ou de la phosphatidyléthanolamine et de l'eau.

15. Procédé selon la revendication 14, caractérisé en ce que les cyclosporines sont totalement dissoutes, avant l'émulsification, dans l'huile d'origine naturelle, et sont ensuite émulsionnées dans l'eau, à l'aide de 3-sn-phosphatidylcholines et/ou de leurs sels alcalins, en une émulsion brute, et le mélange est ensuite homogénéisé en une émulsion, ayant de préférence une taille de particules inférieure à 10 µm, de façon particulièrement préférée inférieure à 2 µm.

16. Procédé pour la préparation de la composition pharmaceutique contenant une (des) cyclosporine(s), pour administration orale, selon une ou plusieurs des revendications précédentes, caractérisé en ce que le mélange de cyclosporine(s), huiles d'origine naturelle, 3-sn-phosphatidylcholine et eau contient déjà 0,01 à 1 % d'acides gras naturels libres et/ou de leurs sels alcalins, et ce mélange est ajusté, avant l'émulsification, à un pH de 5 - 10, de préférence de 7 - 9, au moyen d'une (de) solution(s) alcaline(s).

17. Procédé pour la préparation de la composition pharmaceutique contenant une(des) cyclosporine(s), pour administration orale, selon une ou plusieurs des revendications précédentes, caractérisé en ce que l'on utilise des acides gras et/ou leurs sels alcalins pour ajuster l'émulsion à un pH de 5 -10, de préférence de 7 - 9.

18. Procédé pour la préparation de la composition pharmaceutique contenant une(des) cyclosporine(s), pour administration orale, selon une ou plusieurs des revendications précédentes, caractérisé en ce que, pour la préparation de la composition pharmaceutique, on utilise des solutions pratiquement exemptes d'oxygène.

19. Procédé pour la préparation de la composition pharmaceutique contenant une(des) cyclosporine(s), pour administration orale, selon une ou plusieurs des revendications précédentes, caractérisé en ce que l'on utilise en outre des acides gras saturés et/ou insaturés, et/ou des sels alcalins des acides gras, pharmaceutiquement acceptables.

20. Procédé pour la préparation de la composition pharmaceutique contenant une(des) cyclosporine(s), pour administration orale, selon une ou plusieurs des revendications précédentes, caractérisé en ce que l'on prépare d'abord une émulsion brute, par vigoureuse agitation, et on effectue ensuite l'émulsification finale dans un homogénéisateur à haute pression, sous des pressions comprises entre 100 et 500 bars, l'homogénéisateur à haute pression comportant de préférence trois pistons.

21. Utilisation de la composition pharmaceutique selon une ou plusieurs des revendications précédentes, pour l'administration orale.
